# EUROPEAN PATENT APPLICATION

(11) **EP 1 985 603 A1**
(43) Date of publication of application: **29.10.2008**
(21) Application number: 07107158.3
(22) Date of filing: 27.04.2007
(51) Int. Cl.: C07C 67/31, C07C 69/757, C07C 67/307, C07C 69/75, C07C 51/09, C07C 61/15, C12P 7/40

(54) **Process for preparing difluorocyclohexanoic acid derivatives**

(71) Applicant: SOLVAY (Société Anonyme), 1050 Bruxelles (BE)
(72) Inventor: Wiesenhöfer, Wolfgang, 40885, Ratingen (DE); Buyle, Olivier, 1367, AUTRE-EGLISE (BE)
(74) Representative: Mross, Stefan P.M.

(57) **Abstract**

The present invention relates to a process for preparing a difluorocyclohexanoic acid derivative comprising subjecting an oxocyclohexanoic acid ester to reaction with an activated fluorocarboxylic acid derivative and fluorinating the obtained intermediate to obtain a difluorocyclohexanoic acid ester.

## Description

The present invention relates to a process for preparing difluorocyclohexanoic acid derivatives.

It is known that difluorocyclohexanoic acid derivatives are useful intermediates in the synthesis of pharmaceuticals. In particular, 4,4-difluorocyclohexanoic acid (DFCHCA) is a building block for the anti HIV drug Maraviroc.

Methods for the preparation of such derivatives are already known. For the preparation of 4,4-difluorocyclohexanoic acid esters, a number of synthetical processes have been described.

US 4,792,618 discloses a process comprising formation of a chloro-substituted cyclohexenoate via Diels-Alder reaction and subsequent fluorination of said substituted chlorohexenoate. However, performance of the Diels-Alder reaction is unsatisfactory, as it may lead to by-products which are difficult to separate. An additional shortcoming of this process consists in the difficult access to one of the starting materials, i.e., a suitably substituted 1,3-diene. A process for the preparation of 4,4-difluorocyclohexanoic acid is disclosed in Tetrahedron Letters, 46 (2005) 5005-5007 and involves fluorination of ethyl 4-oxo-cyclohexanoate by means of a sulfur type fluorinating agent, namely DAST (diethylaminosulfur trifluoride). Said fluorination however results in the formation of a 1:1 mixture of the desired difluoro compound and of an inseparable vinyl fluoride by-product. An additional dihydroxylation step of said by-product is required in order to allow isolation of ethyl 4,4-difluorocyclohexanoate, and the overall yield of the process is anyway compromised.

EP 1 364 938 discloses a two steps process applicable for producing 4,4-difluorocyclohexanoic acid esters. In the first step of the process, ethyl 4-oxocyclohexanoate is reacted with phosphorus pentachloride and the resulting product comprises a mixture of ethyl 4,4-dichlorocyclohexanoate and an olefin by-product in a ratio of about 1:2. In the second step, fluorination of ethyl 4,4-dichlorocyclohexanoate yields the target 4,4-difluorocyclohexanoic acid ester. However, the overall yield of the process is poor. In addition, also the second step reaction is not efficient, since the reported yield of the sole fluorination step is low.

JP63054331A discloses a process for the production of 1,1-difluorocyclohexane, wherein cyclohexanone is reacted with trifluoroacetic anhydride and the resulting 1,1-bis(trifluoroacetoxy)cyclohexane is reacted with hydrogen fluoride to obtain the desired product. JP63041443A and JP63044536A disclose single steps of the same process, whereas JP63054332A discloses a process comprising additional oxidation steps to obtain fluorobenzene.

However, none of the above mentioned documents discloses a process for preparing difluorocyclohexanoic acid derivatives.

It was therefore desirable to solve the above discussed problems of the prior art processes. In particular, it was desirable to provide a process for preparing difluorocyclohexanoic acid derivatives, which affords the desired product with high yield and in high purity.

The present invention concerns in consequence a process for preparing a difluorocyclohexanoic acid derivative, which comprises a) reacting an 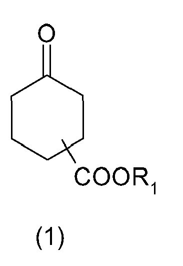 oxocyclohexanoic acid ester of formula
wherein COOR₁ is an ester group
with an activated fluorocarboxylic acid derivative to produce a compound of formula (2), 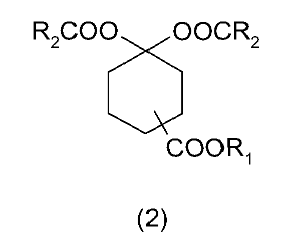 wherein COOR₁ is as defined above and R₂ is a fluorocarbon substituent
and b) reacting said compound of formula (2) with a fluorinating agent to produce a difluorocyclohexanoic acid ester of formula (3), 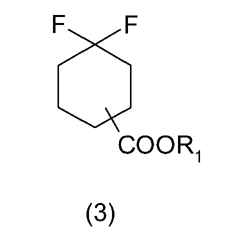 wherein COOR₁ is as defined above.

The process according to the invention especially suitable to produce 4,4-difluorocyclohexanoic acid derivatives (3a) starting from a 4-oxocyclohexanoic acid ester (1a) as shown in the following reaction schema:

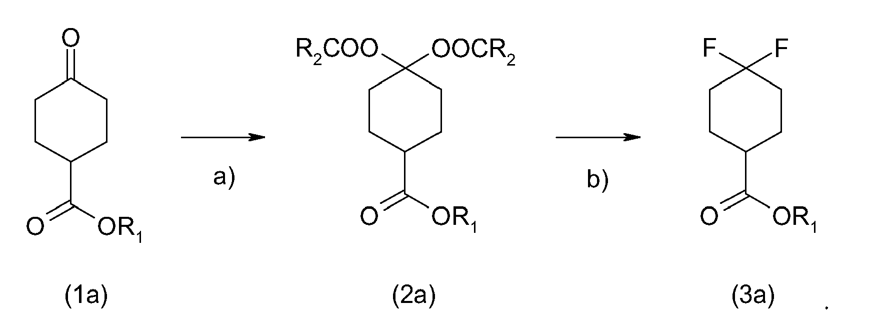

A further embodiment of the present invention concerns a process in which step a) is catalyzed by means of an amine.

A further embodiment of the present invention is a process having an improved efficiency, wherein step a) and b) are carried out one-pot, in a single reaction vessel. In this way, a one-pot preparation of difluorocyclohexanoic acid derivatives is carried out.

A further embodiment of the present invention is a process comprising a step c) of hydrolysis of the difluorocyclohexanoic acid ester recovered from step b), to afford the difluorocyclohexanoic acid. Said hydrolysis can be advantageously performed at neutral pH by using an enzymatic catalyst.

Surprisingly, the process of the present invention allows for selective and high yield manufacture of difluorocyclohexanoic acid derivative. The formation of undesirable by-products can be substantially avoided. Once a reaction of a step is completed, the resulting product can be easily recovered and be used for the next step without or only minimal efforts for purification.

Another advantage of the process according to a preferred embodiment of the present invention consists in that it allows to obtain the desired products in more time efficient manner, due to improved reaction times of step a).

Still another advantage of the process according to the present invention consists in that each step can be carried out under conditions and with materials allowing to avoid e.g. corrosiveness problems.

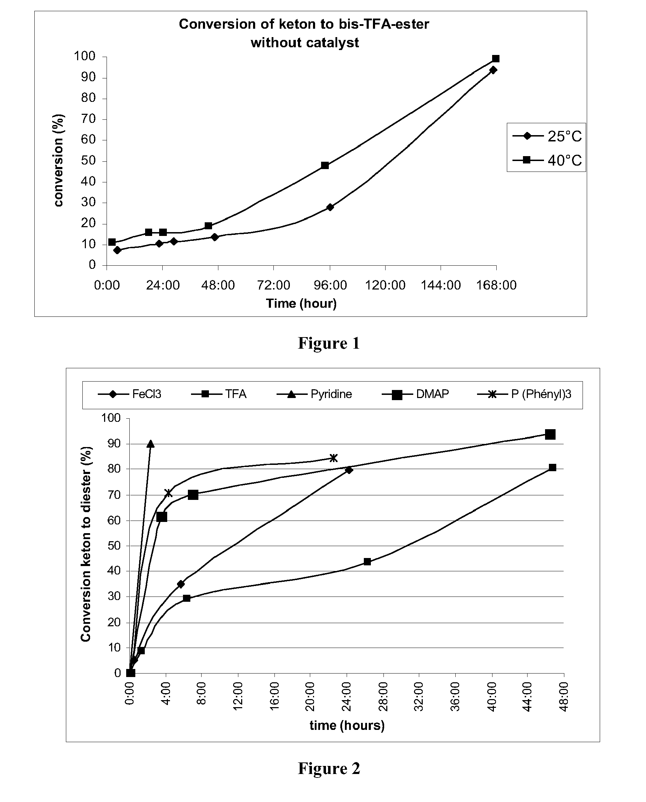

The process according to the present invention will be described in detail in the following, with reference to the attached drawings, wherein:
■ figure 1 shows the performance of the reaction of conversion of ethyl 4-oxocyclohexanoate into ethyl 4,4-bis(trifluoroacetoxy)cyclohexanoate, without catalyst, at 25°C and at 40°C;
■ figure 2 shows the influence of different catalysts on step a) of the reaction;

In the process according to the present invention the substituent R₁ in the ester is generally chosen so that the ester group remains substantially stable when reacted with a fluorinating agent. Examples of such R₁ substituents include hydrocarbon residues such as alkyl, alkaryl or aryl groups which can be optionally be substituted and/or contain catenary or annular heteroatoms such as in particular oxygen atoms. R₁ is preferably selected from substituents consisting of carbon and hydrogen in particular alkyl, alkaryl or aryl substituents containing from 1 to 12, more preferably from 1 to 7 carbon atoms. Most preferably R₁ is selected from methyl, ethyl, propyl or butyl.

The above formula (1), represents cyclohexanoic acid esters bearing the oxo group in the position 2, 3 or 4 of the ring, with respect to the position of the carboxylic function. For example, the process according to the invention is applicable to starting compounds such as 2-oxocyclohexanoic acid esters or 3-oxocyclohexanoic acid esters, in order to prepare 2,2-difluoro or 3,3-difluorocyclohexanoic acid derivatives.

According to a preferred embodiment of the process according to the invention, the starting compound is a 4-oxocyclohexanoic acid ester (1a), so that the resulting products are 4,4-difluorocyclohexanoic acid derivatives.

In step a) of the process, an oxocyclohexanoic acid ester of formula (1) or (1a) is reacted with an activated fluorocarboxylic acid derivative in order to obtain conversion into a bis(fluorocarboxy)cyclohexanoic acid derivative (2) or (2a).

Said activated fluorocarboxylic acid derivative may, for example, be a fluorocarboxylic anhydride or halide, for example a chloride or fluoride, often a chloride. A fluorocarboxylic anhydride is preferred. Often, said activated derivative is selected from derivatives of hydrofluorocarboxylic acids, chlorofluorocarboxylic acids, hydrochlorofluorocarboxylic acids and perfluorocarboxylic acids. It is preferably selected from derivatives of perfluorocarboxylic acids, in particular containing 1 to 10 carbon atoms in the perfluoroalkyl group. It is more preferably selected from derivatives of trifluoroacetic, perfluoropropionic and perfluorobutyric acid.

Even more preferably, said activated derivative is a trifluoroacetic derivative, such as trifluoroacetic chloride, trifluoroacetic fluoride or trifluoroacetic anhydride. Most preferably said activated derivative is trifluoroacetic anhydride. This reagent is particularly advantageous since it has been found that its reaction with oxocyclohexanoic acid esters is extremely selective and leads to the desired product in particularly high yield. In addition, it has the additional advantage that the excess of trifluoroacetic anhydride can be easily removed for example by vacuum evaporation and can be reused in a next batch or recycled to the reaction medium in a continuous process.

It is preferable to apply a molar excess of said activated fluorocarboxylic acid derivative with respect to the oxocyclohexanoic acid ester of formula (1) or (1a). The molar ratio of the activated fluorocarboxylic acid derivative with respect to the oxocyclohexanoic acid ester is preferably from 1.5:1 to 30:1 and more preferably from 2: 1 and 20:1, in particular when trifluoroacetic anhydride is employed.

The reaction of step (a) of the process according to the invention can be carried out in the absence of catalyst.

Figure 1 shows that in this case, when working at 40°C, the complete conversion of the starting material is reached after 168 hours. After the same time, at 25°C, a conversion is of about 90% is observed.

The reaction of step (a) of the process according to the invention can be carried out in the presence of a catalyst. Both acid and basic catalysts are useful.

Acid catalysts can be selected for example from Lewis acids such as for example lewis acid metal salts. Such lewis acid metal salts can be selected from transition metal salts such as iron salts, in particular iron trichloride or ferrous chloride, titanium salts such as titanium tetrachloride. They can also be selected from main group metal salts such as aluminum salts for example aluminium trichloride, antimony salts such as chlorides or tin salts in particular tin chlorides.

Basic catalysts are preferred. It has been found that these catalysts have a significant effect on the reaction kinetics in a large scale. Among these, amine or phosphine catalysts, such as triphenylphosphine [P(Ph)₃], are preferred. Amine catalysts are more preferred. Among the amine catalysts tertiary alkyl amines such as triethylamine or tributylamines and aromatic amines such as pyridine and dimethylaminopyridine [DMAP] give good results. Particularly preferred is pyridine.

Figure 2 shows the results of some tests which were carried out by measuring the time of conversion of ethyl 4-oxocyclohexanoate with trifluoroacetic anhydride at 40°C under the effect of different catalysts.

The catalyst is generally applied in an amount from 0.005 to 0.1 moles per mole of the starting compound. Preferably, the amount of the catalyst equal to or greater than 0.01 moles per mole of the starting compound. Preferably, the amount of the catalyst equal to or lower than 0.05 moles per mole of the starting compound. It has been found, surprisingly, that in particular when the catalyst concentration is in the range described here before, good results can be obtained in step (b) without any need for purification of the product obtained in step (a). Preferred reaction temperatures are from 0°C to 100°C. More preferred are temperatures from 20°C to 60°C.

The pressure can vary. It is preferred to perform the reaction under autogenous pressure.

Surprisingly, in a preferred embodiment the bis(fluorocarboxy)cyclohexanoic acid derivative (2) or (2a), formed in step a) of the process, can be used without any purification for step b).
In step b) of the process, the bis(fluorocarboxy)cyclohexanoic acid derivative (2) or (2a) resulting from step a) is reacted with a fluorinating agent to produce difluorocyclohexanoic acid ester.

Basically any fluorinating agent can be used in step b) of the process, where by fluorinating agents is meant in particular those fluorinating agents in which the fluoride ion serves as a reaction active species.

The preferred fluorinating agent to be used is HF. A reaction with HF is preferably carried out in the substantial absence of catalyst. This embodiment gives particularly good results.

Fluorination catalysts may also be used to catalyze the reaction with the fluorinating agent in particular HF.
Complexes between HF and amines may also be used.

The molar ratio of hydrogen fluoride to the bis(fluorocarboxy)cyclohexanoic acid derivative (2) or (2a) is generally from 2:1 to 200:1. Preferably, said ratio is from 3:1 to 20:1. More preferably, said ratio is from 4:1 to 10:1

In step (b), the reaction temperature is generally from -50°C and 150°C. In a first embodiment, the reaction is advantageously performed at moderate temperatures such as room temperature or temperatures ranging from 10 to 40°C preferably about 25°C. This embodiment allows for particularly selective reaction.

In a second embodiment, the reaction is performed at higher temperatures. Typical temperatures ranging from 40°C to 150°C preferably from 50°C to 100°C. This embodiments, while giving good selectivity allows for increased time-space yield and is particularly useful when the reaction is carried out in continuous mode.

It is preferred to perform the reaction under autogenous pressure. The reaction can also be carried out continuously. In this case, one or more continuously stirred tank reactor, a plug-flow reactor or combinations thereof can be used.

In a continuous process, the residence time defined as the ratio of used volume of the reactor to the volume flow of reactants is generally from 30 minutes to 3 hours and preferably from 60 to 120 minutes. A residence time of from 70 to 100 minutes gives good results.

Generally, in the continuous process, product is withdrawn in a liquid phase. Withdrawal can be carried out, for example, with a dip tube.

A liquid phase obtained in this embodiment can be treated for example, by distillation or evaporation notably of optionally present unreacted HF. Unreacted HF and optional other starting materials recovered can be recycled to the reaction.

The liquid phase obtained in this embodiment can also be treated with an aqueous solution followed by phase separation. Water can be used as aqueous phase. A potassium, sodium or calcium carbonate or hydrogencarbonate solution is preferred as aqeuous solution..

The reactions of steps a) and b) may be carried out in a solvent. Suitable solvents are for example hydrocarbon solvents, ether solvents, halogenated hydrocarbon solvents. Advantageously, the reactions of step (a) and/or step (b) are performed in the absence of solvent.

The above illustrated steps a) and b) of the process may be carried out one-pot. In this case, the oxocyclohexanoic acid ester of formula (1) or (1a), the activated fluorocarboxylic acid derivative and the fluorinating agent are introduced into a single reaction vessel.

Recovery of the difluorocyclohexanoic acid derivative can be carried out by an isolation procedure comprising treating at least a fraction of the reaction mixture from step (b) with an aqueous solution, preferably water or a solution as described above, and subsequently extracting an aqueous fraction obtained from the treatment with the aqueous solution with an organic solvent. After that, the difluorocyclohexanoic acid derivative may be purified by separation methods, such as distillation, as illustrated in example 3.

In a particular embodiment, step (b) is carried out under conditions where hydrolysis or, where possible, transesterification of difluorocyclohexanoic acid ester can occur. This embodiment is suitable in particular when the desired final product is the difluorocyclohexanoic acid.

Since in many cases the final desired product is the free acid, the process may comprise a step c) of hydrolysis of the difluorocyclohexanoic acid ester recovered from step b), to afford the difluorocyclohexanoic acid. In the case that a mixture of difluorocyclohexanoic acid and ester is recovered from the two first steps of the process, then step c) may be optionally carried out on said mixture. Said hydrolysis optionally performed in step c) of the process according to the invention, may be catalysed by acid or basic catalyst.

In a preferred embodiment of the invention, said hydrolysis can be performed by using an enzymatic catalyst advantageously at neutral pH, such as pH 5 to pH 8, preferably about 7.

Enzymes which can be used for the hydrolysis of the difluorocyclohexanoic acid esters include for example Esterase from Bacillus sp., Esterase from Bacillus stearothermophilus, Esterase from Bacillus thermoglucosidasius, Esterase from horse liver, Esterase from Mucor miehei, Esterase from porcine liver, Esterase from rabbit liver, Esterase from Rhizopus oryzae, Esterase from Saccharomyces cerevisiae, Esterase from Thermoanaerobium brockii, Esterase Isoenzyme 1 from hog liver, Esterase Streptomyces diastatochromogenes, Lipase A from Candida antarctica, Cholesterol Esterase from porcine pancreas, Cholesterol Esterase from Pseudomonas fluorescens, Lipase B from Candida antarctica, Lipase from Aspergillus niger, Lipase from Aspergillus oryzae, Lipase from Burkholderia sp., Lipase from Candida cylindracea, Lipase from Candida lipolytica, Lipase from Candida rugosa, Lipase from Chromobacterium viscosum, Lipase from human pancreas, Lipase from Mucor javanicus BioChemika, Lipase from Mucor javanicus, Lipase from Mucor miehei 1, Lipase from Penicillium roqueforti, Lipase from porcine pancreas, Lipase from Pseudomonas cepacia, Lipase from Pseudomonas fluorescens, Lipase from Pseudomonas sp. Type XIII, Lipase from Pseudomonas stutzeri, Lipase from Rhizomucor miehei, Lipase from Rhizopus arrhizus, Lipase from Rhizopus niveus, Lipase from wheat germ, Lipase from Thermomyces lanuginosus, Lipase from Penicillium camemberti, Lipase from Rhizopus oryzae.

Immobilized enzymes are preferred since it has been found that the reaction performs quite rapidly, with very good yields, under mild conditions. The immobilization has also the advantage of easy recycling of the biocatalyst. The difluorocyclohexanoic acid is preferably recovered from the reaction mixture by filtration, and then it is heat-dried.
The invention is described hereinafter be means of examples and figures which serve purely illustrative purposes and in no way limit its scope.

### Example 1: Preparation of ethyl 4,4-bis(trifluoroacetoxy)cyclohexanoate

### Reaction without catalyst at room temperatur and at 40°C.

Ethyl-4-oxocyclohexanoate (84.1g; 0.49mol) and trifluoroacetic anhydride (TFAH: 256.7g, 1.22 mol; 3.47 eq.) were placed in to a 500ml round bottom flask. The solution was stirred at room temperature during 166 hours (an identical trial was run at 40°C during 168 hours). The conversion of the starting compound into ethyl 4,4-bis(trifluoroacetoxy)cyclohexanoate was determined by GC analysis (see Figure 1). After stirring for the mentioned time, the excess of TFAH was removed in a rotavapor (50°C, 40mbar). 187 g were obtained. The composition of the final mixture was the following: 77.7% of ethyl 4,4-bis(trifluoroacetoxy)cyclohexanoate (bis-TFA-ester), 3 % of TFAH and 17 % of ethyl-4-oxocyclohexanoate. The mixture was used without further purification as starting material in a fluorination reaction corresponding to step (b).

### Example 2: Preparation of ethyl 4,4-bis(trifluoroacetoxy)cyclohexanoate

### Reaction with catalyst

Ethyl-4-oxocyclohexanoate (119.6g; 0.703mol), trifluoroacetic anhydride (TFAH: 256.7g, 1.22 mol; 2.47eq) and pyridine (0.57g; 7mmol; 0.01eq) were placed in a 500ml round bottom flask. The solution was stirred at 55°C (reflux) during 45 hours. The conversion of the starting compound into ethyl 4,4-bis(trifluoroacetoxy)cyclohexanoate was determined by GC analysis (see Figure 2). The excess of TFAH was removed in a rotavapor. 290 g were obtained. The composition of the final mixture was the following: 88% of bis-TFA-ester 3.2% of ethyl-4-oxocyclohexanoate, 1.3 % of TFAH. The mixture was used without further purification as starting material in a fluorination reaction corresponding to step (b).

### Example 3: Fluorination of ethyl 4,4-bis(trifluoroacetoxy)cyclohexanoate (Bis-TFA ester) by HF, followed by aqueous work up

All equipments (flask, tube, cooler, bubbler, etc.) were made in PTFE. The bis-TFA-ester (290 g; 0.58 mol) was placed in a 500ml three-neck PTFE round bottom flask equipped with cooler (-10°C), a constant nitrogen flow and tube to introduce the HF. The system was cooled at -30°C before the addition of HF. 116 g of HF (5.8 mol; 7.6 eq.) were added in 25 minutes. The cool bath was removed and the temperature increased slowly to 25°C. It was stirred overnight at room temperature. Afterwards 149 g of water (8.3 mol) were added slowly at 0 °C and an organic phase separated. The organic phase was washed once with water and once with a solution of NaHCO₃. 114 g of organic were obtained, having a content of ethyl 4,4-difluorocyclohexanoate of 85%. The organic phase was distilled and 70.3 g ethyl 4,4-difluorocyclohexanoate with a purity of 98.7 % were obtained.

### Example 4: Fluorination of ethyl 4,4-bis(trifluoroacetoxy)cyclohexanoate (bis-TFA ester) by HF

All equipments (flask, tube, cooler, bubbler, etc.) were made in PTFE. The system was flushed with nitrogen before running the reaction.
The bis-TFA-ester was placed in a 500 ml three-neck PTFE round bottom flask equipped with a cooler (-10°C) and tube to introduce the HF.
The system was cooled to 0°C and HF was added in 30 minutes. The cool bath was removed and the temperature increased slowly to 25°C. Samples were taken by time and analyzed via GC. The quantity of bis-TFA-ester and HF, and the results are summarized in the following table 1.

**Table 1**

| Bis-TFA-ester | | Conversion to ethyl 4,4-difluorocyclohexanoate (time) | | | |
|---|---|---|---|---|---|
| | HF | | | | |
| (g) | Eq. | | | | |
| 182 | 8,74 | 12,6 % | 45,4 % | 62,1 % | 90,7% |
| | | (0:41 h) | (1:55 h) | (6:20 h) | (22:17 h) |

### Example 5: One-pot process

The synthesis was carried out in a Hastelloy C276 reactor of a volume of 250 cc, equipped with a gas turbine, a pressure sensor, a temperature probe, a dipping tube and a rupture disc to 25 b. The reaction temperature was kept at ambient temperature via a water bath. Ethyl 4-oxocyclohexanoate (17.3 g, 0.10 mol) and TFAH (52.9 g, 0.25 mol) were first introduced in the reactor, followed by HF (30.5 g, 1.53 mol) after cooling down the reactor in dry ice and vacuum. Conversions of 50 and 86 % was measured after respectively 18 and 88 h of reaction time. The selectivity to ethyl 4,4-difluorocyclohexanoate reached 90 %.

### Example 6: One-pot process

The synthesis was carried out in the apparatus described in the previous example by introducing consecutively ethyl 4-oxocyclohexanoate (18.6 g, 0.11 mol), the HF (25.0 g, 1.25 mol) and trifluoroacetic chloride (31.0 g, 0.23 mol) after cooling down the reactor and evacuation. The conversion of the ketone was estimated to be 75 % after 16 h of stirring at ambient temperature. The selectivity to ethyl 4,4-difluorocyclohexanoate was 50 %. The main by-products were chlorinated compounds.

### Example 7: Acid-catalyzed hydrolysis of ethyl 4,4-difluorocyclohexanoate

A suspension of ethyl 4,4-difluorocyclohexanoate (41.7 g; 0.22mol) and concentrated aqueous HCl solution (107.5 g; 1.9 mol; 8.8 eq.) was placed into a round bottom flask equipped with a cooler (2°C). The mixture was heated under stirring to 40 °C for 6 h. Afterwards the solution was extracted 5 times with ethyl ether and the combined fractions were washed once with saturated aqueous NaCl solution. The solvent was removed and a solid was obtained which was recrystallised two times from hot chloroform. 19.2 g were isolated with a purity determined by NMR of 99.5% and no olefinic compound was detectable via NMR. The yield was 55%.

### Example 8: Base-catalyzed hydrolysis of ethyl 4,4-difluorocyclohexanoate

A suspension of 1.2 g ethyl 4,4-difluorocyclohexanoate (6.3 mmol), 0.6 g KOH (10.6 mmol) 1.7 ml water and 2.4 g ethanol (52 mmol) was placed into a flask equipped with a cooler (2 °C). The mixture was heated to 40°C for 4h. The largest part of ethanol was removed in vacuum. The residual solid was dissolved in the minimum amount of water. HCl 6M was added until pH=1 was reached. The solution was extracted 3 times with ether. The solvent was removed and 0.5 g of wet solid were obtained. The purity measured by GC, was 100 %.

### Example 9: Enzyme-catalyzed hydrolysis of ethyl 4,4-difluorocyclohexanoate

A suspension of 15.7 g ethyl 4,4-difluorocyclohexanoate (6.3 mmol), 2.1 g Candida antarctica lipase B from Novozyme (NOVO SP 435) and 375 g of buffer (Na₂HPO₄/NaH₂PO₄ pH=7.5, concentration: 50 mM) were placed in a flask. The mixture was stirred at 25°C for 2:30 h. The lipase was filtered off and the solution was acidified until pH=2 by addition of 35g of HCl 6M. A solid was formed and directly filterted. The aqueous phase was 3 times extracted with CH₂Cl₂. The solvent was removed using a rotavapor and 3.5 g of additional solid were obtained. The combined solids were dried. Yield: 74.5%. The purity determined by NMR was 100%.

### Example 10: Reaction at elevated temperature in batch:

In a 500 ml autoclave 181.5 g of bis-TFA-ester and 75 g of HF (9 mol eq.) were introduced at room temperature. The autoclave was then heated under stirring to 90 °C. After 1 h a sample was taken and analyzed by GC. The conversion to 4,4-difluorocyclohexanoate derivatives was quantitative after that time.

### Example 11 -Reaction at elevated temperature in continuous mode:

In a 500 ml autoclave 250 g of bis-TFA-ester and 60 g of HF were introduced at RT. The autoclave was then stirred over night. The next day the temperature was increased to 90 °C and it was stirred for additional 3h. After that time a sample was taken and analyzed by GC. The conversion of that sample was complete. By doing so a start solution for the continuous process was prepared. Afterwards the substrate introduction was started. HF and bis-TFA-ester were introduced continuously via pumps into the reactor. Product was also taken out continuously via a diving tube from the liquid phase. The in and out flows were controlled by balances.

The adjusted bis-TFA-ester flow was: 70 g/h. The adjusted HF flow was: 18 g/h. That corresponds to an HF quantity of 4.5 eq. The adjusted outlet flow was 88 g/h
After running this continuous process for 2:45 h a sample of the outlet flow was taken and analyzed by GC. A conversion to 4,4-difluorocyclohexanoate derivatives of 90 % was observed.

Comparative example 1: Preparation of ethyl 4,4-dichlorocyclohexanoate Solid PCl₅ (109.1 g) was introduced in a three-necked flask provided with a mechanical stirrer and a CaCl₂ cartridge. The flask was cooled down with an ice/water mixture bath and ethyl 4-oxocyclohexanoate (71.6 g) was added slowly. During that time the mixture became homogenous. After stirring overnight and 200g of ice were slowly added. The crude was extracted trice by 200ml of petroleum ether (60-80°C).The organic layer was washed by means of a saturated solution of NaHCO₃ and dried on MgSO₄. The solvent was evaporated in a rotavapor. 63.7 g were isolated and analyzed by GC. The composition of the mixture was the following: 24.6 % of ethyl 4,4-dichlorocyclohexanoate, 72.4 % of 4-chlorocyclohexene ethyl carboxylate. Three other chlorinated species were also identified.

### Comparative example 2: Fluorination

The obtained mixture (63.7 g) of comparative example 1 was used for the fluorination using HF (157.6 g) in a PTFE flask. HF was then introduced at dry ice temperature. The mixture was stirred at room temperature overnight. Afterwards water was added and the occurring phases were separated. The crude was washed three times with 100 ml of water. 20.4 g were obtained and analyzed via GC. The reaction yield of ethyl 4,4-difluorocyclohexanoate was 25%. The above examples and comparative examples demonstrate inter alia that the process according to the present invention affords the desired product with higher yields than the prior art processes.

## Claims

1. Process for preparing a difluorocyclohexanoic acid derivative, which comprises a) reacting an oxocyclohexanoic acid ester of formula 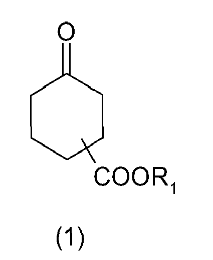 wherein COOR₁ designates an ester group
with an activated fluorocarboxylic acid derivative to produce a compound of formula (2), 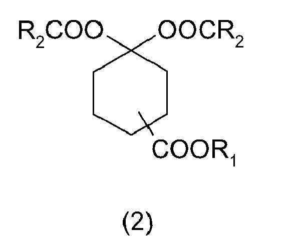 wherein COOR₁ is as defined above and R₂ is a fluorocarbon substituent and b) reacting said compound of formula (2) with a fluorinating agent to produce a difluorocyclohexanoic acid ester of formula (3), 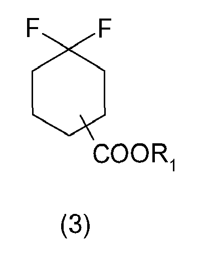 wherein COOR₁ is as defined above.

2. Process according to claim 1, wherein said oxocyclohexanoic acid ester is a 4-oxocyclohexanoic acid ester.

3. Process according to claim 1 or 2, wherein step a) is catalyzed by a nucleophilic catalyst

4. Process according to claim 3, wherein step a) is catalyzed by an amine.

5. Process according to claim 4, wherein said amine is pyridine.

6. Process according to claims 4 or 5, wherein said amine is present in an amount from 0.005 to 0.05 moles per mole of the oxocyclohexanoic acid ester of formula (1).

7. Process according to anyone of claims 1 to 6, wherein said activated fluorocarboxylic acid derivative is selected from activated derivatives of trifluoroacetic acid, trifluoropropionic acid and trifluorobutyric acid.

8. Process according to claim 7, wherein said activated fluorocarboxylic acid derivative is trifluoroacetic anhydride.

9. Process according to anyone of claims 1 to 8, wherein said fluorinating agent is hydrogen fluoride

10. Process according to anyone of claims 1 to 9 wherein step (b) is carried out continuously.

11. Process according to anyone of claims 1 to 10, wherein the difluorocyclohexanoic acid derivative is recovered by an isolation procedure comprising treating at least a fraction of the reaction mixture from step (b) with an aqueous solution, preferably water and subsequently extracting an aqueous fraction obtained from the treatment with the aqueous solution with an organic solvent.

12. Process according to anyone of claims 1 to 10, wherein the difluorocyclohexanoic acid derivative is recovered by a treatment comprising treating at least a fraction of the reaction mixture from step (b) under reduced pressure to reduce the HF content of said fraction.

13. Process according to anyone of claims 1 to 12, wherein the molar ratio between said fluorocarboxylic acid derivative and said oxocyclohexanoic acid ester (1) is from 20: 1 to 2:1.

14. Process according to claim 9, wherein the molar ratio between hydrogen fluoride and said oxocyclohexanoic acid ester of general formula (1) is from 2:1 1 to 20:1.

15. Process according to anyone of claims 1 to 14, wherein steps a) and b) are carried out in one-pot.

16. Process according to anyone of claims 1 to 15, which further comprises c) hydrolysing the difluorocyclohexanoic acid ester to produce the difluorocyclohexanoic acid.

17. Process according to claim 16, wherein said hydrolysis is enzyme catalysed.

18. Process according to claim 17, wherein immobilized enzymes are used.

19. Process according to claim 18, wherein difluorocyclohexanoic acid is recovered from the reaction mixture of step (c) by filtration, preferably after acidification, followed by heat-drying.

20. Compounds of formula (2): 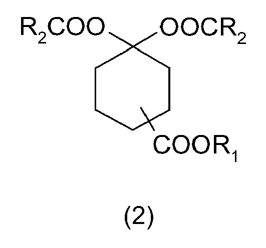 wherein COOR₁ is an ester group and R₂ is a fluorocarbon substituent.

21. Compounds according to claim 20, wherein R₂ is a trifluoromethyl substituent.

22. Compounds according to claim 20 or 21, wherein R₁ is an ethyl substituent.

23. Process for hydrolysing a difluorocyclohexanoic acid ester by using enzymatic catalysis.

24. Process according to claim 23, wherein said catalysis is carried out by means of immobilized enzymes.
